(19)

Europäisches
Patentamt
European
Patent Office
Office européen
des brevets

(11) **EP 4 567 047 A1**

(12) **EUROPEAN PATENT APPLICATION**

(43) Date of publication:
**11.06.2025 Bulletin 2025/24**

(21) Application number: **24212975.7**

(22) Date of filing: **14.11.2024**

(51) International Patent Classification (IPC):
*C07K 16/30* (2006.01)   *C07K 16/18* (2006.01)
*G01N 33/50* (2006.01)

(52) Cooperative Patent Classification (CPC):
**C07K 16/30; C07K 16/18; G01N 33/53**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **17.11.2023 US 202363600401 P**

(71) Applicant: **Uni Pharma Co., Ltd.
Taipei 104 (TW)**

(72) Inventors:
  • **WANG, Yu-Shan
    114 Taipei City (TW)**
  • **CHENG, Chiao-lei
    114 Taipei City (TW)**

  • **LU, Hsiang-Wei
    114 Taipei City (TW)**
  • **LIAO, Ting-En
    114 Taipei City (TW)**
  • **SIO, Ka-Mei
    114 Taipei City (TW)**
  • **KUO, Shan-Shan
    114 Taipei City (TW)**

(74) Representative: **Lang, Christian
    LangPatent Anwaltskanzlei IP Law Firm
    Ingolstädter Straße 5
    80807 München (DE)**

Remarks:
The complete document including Reference
Table(s) and the Sequence Listing(s) can be
downloaded from the EPO website

(54) **RECOMBINANT ANTIBODY, KIT COMPRISING THE SAME, AND USES THEREOF IN DIAGNOSING CANCERS**

(57)    Disclosed herein is a recombinant antibody or a fragment thereof comprising a heavy chain variable (VH) domain and a light chain variable (VL) domain. According to some embodiments of the present disclosure, the VH domain and VL domain of the recombinant antibody or antibody fragment respectively comprise the amino acid sequences at least 85% identical to SEQ ID NOs: 4 and 8. Also disclosed herein are methods for diagnosing whether a subject has a cancer by using the present recombinant antibody or antibody fragment; as well as methods for treating the subject having been diagnosed with the cancer.

**EP 4 567 047 A1**

**Description**

**BACKGROUND OF THE INVENTION**

1. FIELD OF THE INVENTION

The present disclosure in general relates to the field of disease diagnosis. More particularly, the present disclosure relates to a recombinant antibody and uses thereof in diagnosing cancers.

2. DESCRIPTION OF RELATED ART

Cancer refers to a group of diseases characterized by the development of abnormal cells that growth uncontrollably and exhibit the ability to invade and destroy normal tissues. It is a leading cause of death worldwide, accounting for about 10 million deaths every year, or nearly one in six deaths. According to statistics of World Health Organization (WHO), cancer mortality is reduced when cases are detected and treated early, and two strategies of early detection are accordingly suggested by WHO: early diagnosis and screening. Early diagnosis of cancer or precancerous changes allows early intervention to slow or even prevent cancer development, invasion and/or migration, and thus improving the survival rates and life quality of cancer patients, as well as to significantly reducing the cost and complexity of cancer treatment. Screening aims to identify subjects with findings suggestive of cancer or pre-cancer before the subject has developed symptoms.

Tumor-associated antigen (TAA) is useful as a target for cancer screening. In practice, a biological sample (e.g., a blood or biopsy sample) isolated from a subject suspected of having a cancer is subjected to a biomarker test identifying the expression of one or more TAAs in the biological sample, so that a skilled artisan or a clinical practitioner may preliminarily identify cancer cases and optionally perform further tests to establish a definitive diagnosis. In the processes of tumor growth, angiogenesis, invasion and metastasis, cancer cells release plasminogen activators mediating the cleavage of plasminogen to plasmin that then degrades fibrin to fibrin-fibrinogen degradation products (FDPs). It is known that FDPs are produced by all major cancers, including lung cancer, breast cancer, gastric cancer, colorectal cancer, pancreatic cancer, tongue cancer, and hepatocellular carcinoma (HCC). DR-70 is a marker used to measure FDPs, and may serve as a novel target for cancer screening. However, up to the present time, no monoclonal antibody (mAb) is available for the detection of DR-70. In view of the foregoing, there is a continuing interest in developing a novel anti-DR-70 mAb.

SUMMARY

The following presents a simplified summary of the disclosure in order to provide a basic understanding to the reader. This summary is not an extensive overview of the disclosure and it does not identify key/critical elements of the present invention or delineate the scope of the present invention. Its sole purpose is to present some concepts disclosed herein in a simplified form as a prelude to the more detailed description that is presented later.

As embodied and broadly described herein, the first aspect of the present disclosure is directed to a recombinant antibody, designated as "14G12-5", and the fragments thereof (e.g., single-chain variable fragment, scFv). In structure, each of the recombinant antibody and its fragments comprises a heavy chain variable (VH) domain and a light chain variable (VL) domain. According to embodiments of the present disclosure, the VH domain comprises a first complementarity determining region (CDR-H1) of SEQ ID NO: 1, a second complementarity determining region (CDR-H2) of SEQ ID NO: 2, and a third complementarity determining region (CDR-H3) of SEQ ID NO: 3; and the VL domain comprises a first complementarity determining region (CDR-L1) of SEQ ID NO: 5, a second complementarity determining region (CDR-L2) of SEQ ID NO: 6, and a third complementarity determining region (CDR-L3) of SEQ ID NO: 7.

According to some exemplary embodiments, the fragment of the VH domain of the present recombinant antibody has the amino acid sequence of SEQ ID NOs: 9, 10, 11, or 12; while the fragment of the VL domain of the present recombinant antibody has the amino acid sequence of SEQ ID NOs: 13, 14, 15, or 16.

According to some exemplary embodiments, the VH and VL domains of the recombinant antibody/antibody fragment respectively comprise the amino acid sequences at least 85% identical to SEQ ID NOs: 4 and 8; preferably, at least 90% identical to SEQ ID NOs: 4 and 8; more preferably, at least 95% identical to SEQ ID NOs: 4 and 8. In one exemplary example of the present disclosure, the VH and VL domains of the recombinant antibody/antibody fragment respectively comprise the amino acid sequences of SEQ ID NOs: 4 and 8 (*i.e.*, the VH and VL domains respectively comprise the amino acid sequences 100% identical to SEQ ID NOs: 4 and 8).

The second aspect of the present disclosure pertains to the use of the present recombinant antibody or antibody fragment in the preparation of a kit for detecting DR-70 (FDP). According to some embodiments of the present disclosure, the kit comprises a first container for housing the recombinant antibody 14G12-5 or its fragment (*e.g.*, 14G12-5 scFv) therein, and a second container for housing a pharmaceutically acceptable carrier therein.

Also disclosed herein is a method of making a diagnosis on whether a subject has a cancer via use of a biological sample isolated from the subject. The method comprises mixing the biological sample with the present recombinant antibody or a fragment thereof so as to react DR-70 in the biological sample with the recombinant antibody of claim 1 or the fragment thereof thereby forming a complex in an immunological assay, wherein the formation of the complex is an indication that the subject has the cancer.

Alternatively, or optionally, the method further comprises adding a secondary antibody to detect the complex formed in step (ii), wherein the secondary antibody comprises a VH domain of SEQ ID NO: 20 and a VL domain of SEQ ID NO: 24.

According to embodiments of the present disclosure, the cancer is lung cancer, breast cancer, gastric cancer, colorectal cancer, pancreatic cancer, tongue cancer, or HCC.

According to embodiments of the present disclosure, the subject is a mammal; preferably, a human.

Many of the attendant features and advantages of the present disclosure will becomes better understood with reference to the following detailed description.

## DETAILED DESCRIPTION OF THE INVENTION

The detailed description provided below is intended as a description of the present examples and is not intended to represent the only forms in which the present example may be constructed or utilized. The description sets forth the functions of the example and the sequence of steps for constructing and operating the example. However, the same or equivalent functions and sequences may be accomplished by different examples.

## I. DEFINITION

For convenience, certain terms employed in the specification, examples and appended claims are collected here. Unless otherwise defined herein, scientific and technical terminologies employed in the present disclosure shall have the meanings that are commonly understood and used by one of ordinary skill in the art. Also, unless otherwise required by context, it will be understood that singular terms shall include plural forms of the same and plural terms shall include the singular. Specifically, as used herein and in the claims, the singular forms "a" and "an" include the plural reference unless the context clearly indicates otherwise. Also, as used herein and in the claims, the terms "at least one" and "one or more" have the same meaning and include one, two, three, or more.

Notwithstanding that the numerical ranges and parameters setting forth the broad scope of the invention are approximations, the numerical values set forth in the specific examples are reported as precisely as possible. Any numerical value, however, inherently contains certain errors necessarily resulting from the standard deviation found in the respective testing measurements. Also, as used herein, the term "about" generally means within 10%, 5%, 1%, or 0.5% of a given value or range. Alternatively, the term "about" means within an acceptable standard error of the mean when considered by one of ordinary skill in the art. Other than in the operating/working examples, or unless otherwise expressly specified, all of the numerical ranges, amounts, values and percentages such as those for quantities of materials, durations of times, temperatures, operating conditions, ratios of amounts, and the likes thereof disclosed herein should be understood as modified in all instances by the term "about". Accordingly, unless indicated to the contrary, the numerical parameters set forth in the present disclosure and attached claims are approximations that can vary as desired. At the very least, each numerical parameter should at least be construed in light of the number of reported significant digits and by applying ordinary rounding techniques.

The term "antibody" (Ab) is used in the broadest sense and specifically covers monoclonal antibodies (including full length monoclonal antibodies), polyclonal antibodies, multi-specific or multivalent antibodies (e.g., bi-specific antibodies), chimeric antibodies, humanized antibodies and antibody fragments so long as they exhibit the desired biological activity. The term "antibody fragment" or "the fragment of an antibody" refers to a portion of a full-length antibody, generally the antigen binding or variable domain (*i.e.*, VH and VL domains) of a full-length antibody. Examples of the antibody fragment include fragment antigen-binding (Fab), Fab', F(ab')$_2$, single-chain variable fragment (scFv), diabody, linear antibody, single-chain antibody molecule, and multi-specific antibody formed from antibody fragments. Depending on the antibody amino acid sequence of the constant domain of its heavy chains, immunoglobulins can be assigned to different classes, including immunoglobulin G (IgG), immunoglobulin A (IgA), immunoglobulin M (IgM), immunoglobulin D (IgD) or immunoglobulin E (IgE), in which some classes may be further divided into subclasses (isotypes), such as IgG1, IgG2, IgG3, IgG4, IgA1 and IgA2. The heavy-chain constant domains that correspond to the different classes of immunoglobulins are called alpha ($\alpha$), gamma ($\gamma$), delta ($\delta$), epsilon ($\varepsilon$), and mu ($\mu$), respectively. The subunit structures and three-dimensional configurations of different classes of immunoglobulins are well known in the art, for example, Abbas et al., "Cellular and Molecular Immunology," 4th ed. (2000). An antibody may be part of a larger fusion molecule, formed by covalent or non-covalent association of the antibody with one or more other proteins or peptides.

As used herein, the term "monoclonal antibody" (mAb) refers to an antibody obtained from a population of substantially homogeneous antibodies. In contrast to polyclonal antibodies which may include different antibodies directed to different epitopes, each monoclonal antibody is directed against a single determinant (*i.e.,* epitope) on the antigen. Monoclonal antibodies may be produced by fusing a normally short-lived, antibody-producing B cell to a fast-growing cell, such as an immortal cell. The resulting hybrid cell, or hybridoma, multiplies rapidly, creating a clone that produces large quantities of the antibody. Alternatively, monoclonal antibodies may also be produced by recombinant DNA methods, in which a portion of the heavy and/or light chain is identical with or homologous to corresponding sequences in antibodies derived from a species or belonging to an antibody class or subclass, while the remainder of the chain identical with or homologous to corresponding sequences in antibodies derived from another species or belonging to another antibody class or subclass, as well as fragments of such antibodies, as long as they exhibit the desired biological activity.

As used herein, the term "recombinant antibody" refers to an antibody that is expressed and isolated from a cell or cell line transfected with an expression vector (or possibly more than one expression vector, typically two expression vectors) comprising the coding sequence of the antibody, where said coding sequence is not naturally associated with the cell.

The "variable domain" of an antibody refers to the amino-terminal domains of heavy or light chain of the antibody. These domains are generally the most variable parts of an antibody and contain the antigen-binding sites. The term "variable" refers to the fact that certain portions of the variable domains differ extensively in sequence among antibodies and are used in the binding and specificity of each particular antibody for its particular antigen. However, the variability is not evenly distributed throughout the variable domains of antibodies. It is concentrated in three segments called complementarity-determining regions (CDRs) or hypervariable regions both in the light-chain and the heavy-chain variable domains. The more highly conserved portions of variable domains are called the framework (FR). The variable domains of native heavy and light chains each comprise four FR regions, largely adopting a beta-sheet configuration, connected by three CDRs, which form loops connecting, and in some cases forming part of, the beta-sheet structure. The CDRs in each chain are held together in close proximity by the FR regions and, with the CDRs from the other chain, contribute to the formation of the antigen-binding site of antibodies (see Kabat et al., Sequences of Proteins of Immunological Interest, Fifth Edition, National Institute of Health, Bethesda, Md. (1991)).

As discussed herein, minor variations in the amino acid sequences of antibodies (especially minor variations in the FR sequences of antibodies) are contemplated as being encompassed by the presently disclosed and claimed inventive concept(s), providing that the variations in the amino acid sequence maintain at least 85% sequence identity, such as at least 85%, 86%, 87%, 88%, 89%, 90%, 91%, 92%, 93%, 94%, 95%, 96%, 97%, 98% and 99% sequence identity. The antibody of the present disclosure may be modified specifically to alter a feature of the antibody unrelated to its physiological activity. For example, certain amino acid residues in the framework (FR) region of the antibody can be changed and/or deleted without affecting the physiological activity of the antibody in this study (*i.e.,* its ability to treat cancers). In particular, conservative amino acid replacements are contemplated. Conservative replacements are those that take place within a family of amino acid residues that are related in their side chains. Genetically encoded amino acid residues are generally divided into families: (1) acidic = aspartate, glutamate; (2) basic = lysine, arginine, histidine; (3) nonpolar = alanine, valine, leucine, isoleucine, proline, phenylalanine, methionine, tryptophan; and (4) uncharged polar = glycine, asparagine, glutamine, cysteine, serine, threonine, tyrosine. More preferred families are: serine and threonine are aliphatic-hydroxy family; asparagine and glutamine are an amide-containing family; alanine, valine, leucine and isoleucine are an aliphatic family; and phenylalanine, tryptophan, and tyrosine are an aromatic family. For example, it is reasonable to expect that an isolated replacement of a leucine with an isoleucine or valine, an aspartate with a glutamate, a threonine with a serine, or a similar replacement of an amino acid with a structurally related amino acid will not have a major effect on the binding or properties of the resulting molecule, especially if the replacement does not involve an amino acid residue within the antigen-biding sites, *i.e.,* CDRs. Whether an amino acid change results in a functional peptide can readily be determined by assaying the specific activity of the peptide derivative. Fragments or analogs of proteins/peptides can be readily prepared by those of ordinary skill in the art. Preferred amino- and carboxy-termini of fragments or analogs occur near boundaries of functional domains.

"Percentage (%) sequence identity" is defined as the percentage of amino acid residues in a candidate sequence that are identical with the amino acid residues in the specific peptide sequence, after aligning the sequences and introducing gaps, if necessary, to achieve the maximum percent sequence identity, and not considering any conservative substitutions as part of the sequence identity. Alignment for purposes of determining percentage sequence identity can be achieved in various ways that are within the skill in the art, for instance, using publicly available computer software such as BLAST, BLAST-2, ALIGN or Megalign (DNASTAR) software. Those skilled in the art can determine appropriate parameters for measuring alignment, including any algorithms needed to achieve maximal alignment over the full length of the sequences being compared. For purposes herein, sequence comparison between two amino acid sequences was carried out by computer program Blastp (protein-protein BLAST) provided online by Nation Center for Biotechnology Information (NCBI). The percentage amino acid sequence identity of a given amino acid sequence A to a given amino acid sequence B (which can alternatively be phrased as a given amino acid sequence A that has a certain % amino acid sequence identity to a given amino acid sequence B) is calculated by the formula as follows:

$$\frac{X}{Y} \times 100$$

where X is the number of amino acid residues scored as identical matches by the sequence alignment program BLAST in that program's alignment of A and B, and where Y is the total number of amino acid residues in A or B, whichever is shorter.

The phrase "pharmaceutically acceptable" refers to molecular entities and compositions that are "generally regarded as safe," *e.g.*, that are physiologically tolerable and do not typically produce an allergic or similar untoward reaction, such as gastric upset, dizziness and the like, when administered to a human. Preferably, as used herein, the term "pharmaceutically acceptable" means approved by a regulatory agency of the Federal or a state government or listed in the U.S. Pharmacopeia or other generally recognized pharmacopeia for use in animals, and more particularly in humans.

As used herein, the term "detecting" or "detection", in addition to art-recognized meanings, means any process of observing a marker, in a biological sample, whether or not the marker is actually detected. In other words, the act of probing a sample for a marker is a "detection" even if the marker is determined to be not present or below the level of sensitivity. Detection may be a quantitative, semi-quantitative or non-quantitative observation.

The term "diagnosis" as used herein refers to methods by which a skilled artisan can estimate and/or determine the probability ("a likelihood") of whether or not a patient is suffering from a given disease or condition (*e.g.,* a cancer). In the case of the present invention, "diagnosis" includes using the detection results of the present invention (*i.e.,* the presence or absence of DR-70), optionally together with other clinical characteristics, to arrive at a diagnosis (that is, the occurrence or nonoccurrence) of a cancer for the subject from which a sample was obtained and assayed. That such a diagnosis is "determined" is not meant to imply that the diagnosis is 100% accurate. Different biomarkers are indicative of multiple conditions. A skilled clinician does not use single one biomarker result in an informational vacuum, but rather the test result is used together with other clinical indicia to arrive at a diagnosis. Thus, a measured biomarker level on one side of a predetermined diagnostic threshold indicates a greater likelihood of the occurrence of disease in the subject relative to a measured level on the other side of the predetermined diagnostic threshold.

The term "subject" refers to an animal. Preferably, the animal is a mammal, for example, a human, a cow, a goat, a sheep, a horse, a dog, a cat, a rabbit, a monkey, a rat, or a mouse. The term "subject" is intended to refer to both the male and female gender unless one gender is specifically indicated.

## II. DESCRIPTION OF THE INVENTION

### *(i) Recombinant antibody*

The subject invention aims at providing a novel antibody for detecting DR-70 (FDP), as well as kits for such purpose.

Accordingly, the first aspect of present disclosure provides a recombinant mAb designated as "14G12-5". According to embodiments of the present disclosure, the mAb 14G12-5 comprises a heavy chain variable (VH) domain and a light chain variable (VL) domain. The VH domain comprises a first complementarity determining region (CDR-H1) of SEQ ID NO: 1, a second complementarity determining region (CDR-H2) of SEQ ID NO: 2, and a third complementarity determining region (CDR-H3) of SEQ ID NO: 3; and the VL domain comprises a first complementarity determining region (CDR-L1) of SEQ ID NO: 5, a second complementarity determining region (CDR-L2) of SEQ ID NO: 6, and a third complementarity determining region (CDR-L3) of SEQ ID NO: 7.

According to some optional embodiments, the VH domain of mAb 14G12-5 comprises the amino acid sequence at least 85% (*i.e.,* 85%, 86%, 87%, 88%, 89%, 90%, 91%, 92%, 93%, 94%, 95%, 96%, 97%, 98%, 99% or 100%) identical to SEQ ID NO: 4, and the VL domain of mAb 14G12-5 comprises the amino acid sequence at least 85% (*i.e.,* 85%, 86%, 87%, 88%, 89%, 90%, 91%, 92%, 93%, 94%, 95%, 96%, 97%, 98%, 99% or 100%) identical to SEQ ID NO: 8. According to some preferred embodiments, the VH and VL domains of mAb 14G12-5 respectively comprise the amino acid sequences at least 90% identical to SEQ ID NOs: 4 and 8. More preferably, the VH and VL domains of mAb 14G12-5 respectively comprise the amino acid sequences at least 95% identical to SEQ ID NOs: 4 and 8. Most preferably, the VH and VL domains of mAb 14G12-5 respectively comprises the amino acid sequences 100% identical to SEQ ID NOs: 4 and 8.

Since the binding affinity and specificity of an antibody are mainly determined by the CDR sequences thereof, as could be appreciated, the framework (FR) sequences of the VH and VL domains may vary (e.g., being substituted by conserved or non-conserved amino acid residues) without affecting the binding affinity and/or specificity of the present antibody. Preferably, the FR sequence is conservatively substituted by one or more suitable amino acid(s) with similar properties; for example, the substitution of leucine (an nonpolar amino acid residue) by isoleucine, alanine, valine, proline, phenylalanine, or tryptophan (another nonpolar amino acid residue); the substitution of aspartate (an acidic amino acid residue) by glutamate (another acidic amino acid residue); or the substitution of lysine (an basic amino acid residue) by arginine or histidine (another basic amino acid residue).

Based on the conservative substitution, a skilled artisan may substitute the amino acid residue(s) of the FR sequences of the VH and VL domains of mAb 14G12-5 without affecting its activity and/or effect (*i.e.,* binding to DR-70 and/or diagnosing cancers). Accordingly, the antibody comprising substituted amino acid(s) in its FR sequences of VH and VL domains are intended to be included within the scope of the present disclosure. Accordingly, fragments to the VH or VL domains of mAb 14G12-5 are created. According to some embodiments of the present disclosure, the fragment of the VH domain has the amino acid sequence of SEQ ID NOs: 9, 10, 11, or 12. According to other embodiments of the present disclosure, the fragment of the VL domain has the amino acid sequence of SEQ ID NOs: 13, 14, 15, or 16.

Depending on intended purpose, the present mAb 14G12-5 may be produced in the form of IgG, IgA, IgM, IgD or IgE. According to some exemplary embodiments, the present mAb is produced in the form of IgG.

Also disclosed herein are the fragments of the present mAb, including scFv, Fab, Fab', $F(ab')_2$, and diabody. According to some embodiments, the antibody fragment is scFv.

The present mAb and antibody fragment may be produced by any methods known in the art, for example, phage-displayed libraries, conventional immunization method (*i.e.,* immunizing animals with a specific peptide to induce the animal producing peptide-specific Ab), or recombinant DNA technology (also known as DNA cloning technology; *i.e.,* constructing and transducing a recombinant DNA encoding a specific Ab into a host cell thereby expressing the Ab). Preferably, the present mAb 14G12-5 are produced by recombinant DNA technology, in which the nucleic acids encoding the CDR sequences and/or VH and VL sequences are cloned into an expression vector (*e.g.,* IgG expression vector), followed by transfecting the expression vector into a suitable host cell (preferably, a mammalian cell, *e.g.,* HEK293 cell) so as to produce the present mAb from the host cell. The procedures for carrying out the recombinant DNA technique are known by the person having ordinary skill in the art; hence, the detailed description is omitted herein, for the sake of brevity.

### *(ii) Kits comprising the present mAb or antibody fragment*

According to certain embodiments of the present disclosure, the present mAb 14G12-5 exhibits a binding affinity to DR-70. The second aspect of the present disclosure is thus directed to a kit for detecting DR-70. According to some embodiments, the present kit comprises a first container containing the mAb 14G12-5 or its fragment (*e.g.,* 14G12-5 scFv) as described in *Section (i)* of the present disclosure, and a second container containing a pharmaceutically acceptable carrier.

The pharmaceutically acceptable carrier may be a solvent, dispersion agent, antibacterial agent, antifungal agent, isotonic agent, or other agents that are physiologically compatible. Examples of the pharmaceutically acceptable carrier suitable for use in the present kit include, but are not limited to, water, saline, phosphate buffered saline (PBS), tris-buffered saline (TBS), glycerol, ethanol and a combination thereof. The pharmaceutically acceptable carrier may further comprise minor amounts of auxiliary substances such as wetting or emulsifying agents, preservatives or buffers, which enhance the stability or effectiveness of antibody, or antigen-binding portion thereof.

In practice, the anti-DR-70 antibody (*e.g.,* mAb 14G12-5 or 14G12-5 scFv) of the present kit is employed as a detection or capture antibody for detecting DR-70 expression. Depending on desired purposes, the present kit may further comprise a second anti-DR-70 antibody, a blocking agent (*i.e.,* the agent for blocking or minimizing non-specific binding between antigen and antibody), and/or a reporter (*e.g.,* a fluorescent molecule or horseradish peroxidase (HRP)) conjugated secondary antibody (*e.g.,* anti-mouse, anti-rat, anti-rabbit, or antihuman antibody, in accordance with intended use). For example, in the case when the expression level of DR-70 in a sample is determined by the present kit via western blotting assay, flow cytometry assay, immunochemistry assay or immunofluorescence assay, then the present kit may comprise the present anti-DR-70 antibody (*e.g.,* mAb 14G12-5 or 14G12-5 scFv) as the first antibody for detecting DR-70, the reporter conjugated anti-mouse antibody as the second antibody for detecting the first antibody (*i.e.,* the present anti-DR-70 antibody), and optionally, the blocking agent. Alternatively, when the present kit is employed to detect the expression level of DR-70 via enzyme-linked immunosorbent assay (ELISA), then it may comprise the present anti-DR-70 antibody (*e.g.,* mAb 14G12-5 or 14G12-5 scFv) as the capture antibody or the detection antibody, the second anti-DR-70 antibody as the detection antibody (in the case when the present anti-DR-70 antibody is used as the capture antibody) or the capture antibody (in the case when the present anti-DR-70 antibody is used as the detection antibody), and optionally, the blocking agent. In this case, the detection antibody is preferably conjugated with a reporter for the detection purpose.

### *(iii) Uses of the present antibody or kit for diagnosing cancers*

The third aspect of the present disclosure pertains to a method of making a diagnosis of cancer by using the present antibody or kit. The method comprises, detecting the presence or absence of DR-70 in a biological sample isolated from the subject by mixing the biological sample with the present recombinant antibody or a fragment thereof so as to react DR-70 in the biological sample with the recombinant antibody or the fragment thereof thereby forming a complex in an immunological assay, in which the formation of the complex is an indication that the subject has the cancer.

Alternatively, or optionally, the method further comprises adding a secondary antibody to detect the complex, wherein the secondary antibody comprises a VH domain of SEQ ID NO: 20 and a VL domain of SEQ ID NO: 24.

Preferably, the subject suitable to be diagnosed by the present method is a mammal, for example, a human, cow, goat, sheep, horse, dog, cat, rabbit, monkey, rat, or mouse. More preferably, the subject is a human.

Depending on intended use, the biological sample may be a whole blood sample, a plasma sample, a serum sample, or a biopsy sample. According to some preferred embodiments, the biological sample is the serum sample.

According to embodiments of the present disclosure, the presence or absence of DR-70 in the biological sample is detected by using the present recombinant antibody (*e.g.*, mAb 14G12-5 or 14G12-5 scFv), in which a complex between DR-70 and the present recombinant antibody is formed in an immunological assay, for example, ELISA, flow cytometry assay, western blotting assay, immunochemistry assay and etc.

According to embodiments of the present disclosure, in the case when the level of DR-70 in the biological sample is higher than that of DR-70 in a control sample isolated from a healthy subject or a reference sample (for example, the reference data obtained from gene/protein expression databases that are normalized to allow direct quantitative comparison with the data from the test sample), then the subject has the cancer. By contrast, in the case when the level of DR-70 in the biological sample equals to or is lower than that of DR-70 in a control sample isolated from a healthy subject or a reference sample, then the subject does not have the cancer.

Alternatively, the presence of DR-70 in the biological sample may be regarded as a positive screening result, and a skilled artisan or a practitioner may preliminarily identify cancer cases and optionally perform further tests to establish a definitive diagnosis.

According to certain alternative embodiments, the presence of DR-70 in the biological sample indicates that the subject is at a high risk of having the cancer, and a further test may be required to make a definitive diagnosis, for example, computerized tomography (CT) scan, bone scan, magnetic resonance imaging (MRI), positron emission tomography (PET) scan, ultrasound, X-ray, biopsy, urine and blood test, and/or physical examination. On the contrary, the absence of DR-70 in the biological sample indicates that the subject is at a low risk of having a cancer.

According to some alternative embodiments, in the case when the expression level of DR-70 in the biological sample is higher than that of DR-70 in a control sample isolated from a healthy subject or a reference sample, then the subject is at a high risk of having the cancer, and a further test may be required to make a definitive diagnosis, for example, CT scan, bone scan, MRI, PET scan, ultrasound, X-ray, biopsy, urine and blood test, and/or physical examination. By contrast, in the case when the expression level of DR-70 in the biological sample equals to or is lower than that of DR-70 in a control sample isolated from a healthy subject or a reference sample, then the subject is at a low risk of having a cancer.

The cancer suitable to be diagnosed via the present method may be lung cancer, breast cancer, gastric cancer, colorectal cancer, pancreatic cancer, tongue cancer, HCC, or any cancers producing or associated with FDP, *e.g.*, ovarian cancer, cervical cancer, lymphoma, nasopharyngeal carcinoma (NPC), and esophageal cancer.

Based on the diagnostic results, the subject may then be subjected to a suitable anticancer treatment, including, but not limiting to, surgery, chemotherapy, radiotherapy, hormone therapy, targeted therapy, immunotherapy or anti-angiogenic therapy, to remove the cancer or suppress the growth of the cancer.

The following Examples are provided to elucidate certain aspects of the present invention and to aid those of skilled in the art in practicing this invention. These Examples are in no way to be considered to limit the scope of the invention in any manner. Without further elaboration, it is believed that one skilled in the art can, based on the description herein, utilize the present invention to its fullest extent. All publications cited herein are hereby incorporated by reference in their entirety.

**EXAMPLE**

**Materials and Methods**

The present antibody was designated as "mAb 14G12-5", and the amino acid sequences of which was summarized in Table 1 below.

Table 1 Amino acid sequences of the present mAb 14G12-5 and fragments thereof

| Name | Sequence | SEQ ID NO |
|---|---|---|
| 14G12-5_ CDR-H1 | SGFAFNAYDM | 1 |
| 14G12-5_ CDR-H2 | ISSSGDYT | 2 |
| 14G12-5_ CDR-H3 | VYYHYDEGLDY | 3 |

(continued)

| Name | Sequence | SEQ ID NO |
|------|----------|-----------|
| 14G12-5_VH | MSTEHRHLTMNFGLRLIFLVLTLKGVKCEVQLVESGGGL VKPGGSLKLSCAASGFAFNAYDMSWVRQTPEKRLEWVA YISSSGDYTYYPDTVKGRFTVSRDNAKSTLYLHMGSLKS EDTAMYYCAVYYHYDEGLDYWGQGTALTVSSA | 4 |
| 14G12-5_ CDR-L1 | ASSSVNYMH | 5 |
| 14G12-5_ CDR-L2 | YDTSKLAS | 6 |
| 14G12-5_ CDR-L3 | QQWSSNPPT | 7 |
| 14G12-5_VL | MDFQVQIFSFLLISASVIISRGQIVLIQSPAIMSASPGEKVT MTCSASSSVNYMHWYQQKSGTSPKRWIYDTSKLASGVP ARFSGSGSGTSYSLTISSMEAEDAATYYCQQWSSNPPTFG GGTKLEIKR | 8 |
| 14G12-5_VH FR1 | EVQLVESGGGLVKPGGSLKLSCAA | 9 |
| 14G12-5_VH FR2 | SWVRQTPEKRLEWVAY | 10 |
| 14G12-5_VH FR3 | YYPDTVKGRFTVSRDNAKSTLYLHMGSLKSEDTAMYYC A | 11 |
| 14G12-5_VH FR4 | WGQGTALTVSSA | 12 |
| 14G12-5_VL FR1 | QIVLIQSPAIMSASPGEKVTMTCS | 13 |
| 14G12-5_VL FR2 | WYQQKSGTSPKRWI | 14 |
| 14G12-5_VL FR3 | GVPARFSGSGSGTSYSLTISSMEAEDAATYYC | 15 |
| 14G12-5_VL FR4 | FGGGTKLEIKR | 16 |

The amino acid sequences of a detection antibody (or secondary antibody) designated as "mAb 31H11-2" were summarized in Table 2 below.

Table 2. Amino acid sequences of the mAb 31H11-2

| Name | Sequence | SEQ ID NO |
|------|----------|-----------|
| 31H11-2_ CDR-H1 | GFTFSRSTMS | 17 |
| 31H11-2_ CDR-H2 | TISSGGGFT | 18 |
| 31H11-2_ CDR-H3 | TKYGSYVGYFDV | 19 |
| 31H11-2 VH | MSSEHRPLTMNFGLRLIFLVLTLKGVQCDVKLVESGGGL VKPGGSLKLSCAASGFTFSRSTMSWVRQTPEKRLEWVA TISSGGGFTYYPDTVRGRFTISRDNDKNTLYLQMSSLKS EDTAMYFCTKYGSYVGYFDVWGAGTTVTVSSA | 20 |
| 31H11-2_ CDR-L1 | TASSSVSSSYLH | 21 |
| 31H11-2_ CDR-L2 | STSNLSS | 22 |
| 31H11-2_ CDR-L3 | HQYQRSPWT | 23 |
| 31H11-2_VL | MDFQVQIFSFLLISASVIMSRGQIVLTQSPAIMSTSLGERV TMTCTASSSVSSSYLHWYQQKPGSSPKLWMYSTSNLSS | 24 |

(continued)

| Name | Sequence | SEQ ID NO |
|---|---|---|
| | GVPARFSGSGSGTSYSLTISSMEAEDAATYYCHQYQRSP WTFGGGTKLEIK | |

*Enzyme-linked immunosorbent assay (ELISA)*

96-well ELISA plates were coated with mAb 14G12-5 (48 ng/well) in sodium borate buffer (pH 8.5) and incubated at 20-25°C overnight (at least 16 hours), followed by washing with sodium borate buffer (pH 8.5) at room temperature for 3 times. Blocking buffer (StabilCoat™) was added to the plates, and then incubated at 20-25°C overnight (at least 16 hours). After removing the blocking buffer and washing with PBS-T (PBS containing 0.1% TWEEN®-20) for 3 times, different concentrations of serum sample were added to the wells. The plates were incubated at 22-28°C for 30 minutes, followed by washing with PBS-T for 6 times. HRP-conjugated mAb 31H11-2 (24 ng/well; that has the VH sequence of SEQ ID NO: 20 and the VL sequence of SEQ ID NO: 24) was added to the plates. The plates were incubated at 22-28°C for 30 minutes. After three washes with PBS-T, the signal was developed using 3, 3', 5, 5'-tetramethylbenzidine (TMB) substrate. The reaction was stopped with TMB stop buffer, and absorbance was measured at 450 nm by an ELISA reader.

**Example 1 Characterization of mAb 14G12-5**

The binding activity of the present mAb 14G12-5 to human FDP was first examined. According to the result, mAb 14G12-5 exhibited a binding affinity to human FDP, with an $EC_{50}$ of 30.1 ng/$\mu$l (about $2 \times 10^{-7}$ M) (data not shown). The kinetics and binding affinity of mAb 14G12-5 toward canine FDP or human FDP were respectively summarized in Tables 3 and 4.

Table 3 Kinetic analysis of mAb 14G12-5

| 14G12-5 mAb Kinetic 1:1 Binding | | | | | |
|---|---|---|---|---|---|
| Sample | Ka (1/Ms) | Kd (1/s) | KD (M) | Rmax (RU) | $Chi^2$ ($RU^2$) |
| Canine FDP | $4712 \times 10^4$ | $2.6 \times 10^{-3}$ | $5.524 \times 10^{-8}$ | 42.4 | 2.14 |
| Human FDP | $1.363 \times 10^5$ | $2.25 \times 10^{-3}$ | $1.653 \times 10^{-8}$ | 536.8 | 28.2 |
| Ka: Association rate constant. Kd: Dissociation rate constant. KD: Equilibrium dissociation constant. Rmax: Analyte binding capacity of the surface. $Chi^2$ : a measure of the average squared residual (the difference between the experimental data and the fitted curve). | | | | | |

Table 4 Binding affinity of mAb 14G12-5 toward canine FDP or human FDP

| 14G12-5 mAb Affinity test | | | |
|---|---|---|---|
| Sample | KD (M) | Rmax (RU) | $Chi^2$ ($RU^2$) |
| Canine FDP | $5.032 \times 10^{-6}$ | 975.7 | 0.124 |
| Human FDP | $1.567 \times 10^{-7}$ | 878.5 | 0.500 |
| KD: Equilibrium dissociation constant. Rmax: Analyte binding capacity of the surface. $Chi^2$ : a measure of the average squared residual (the difference between the experimental data and the fitted curve). | | | |

The data of Tables 3 and 4 demonstrated the binding affinity of mAb 14G12-5 toward FDP, in which the binding capacity of mAb 14G12-5 for human FDP was 10-fold higher than that of mAb 14G12-5 for canine FDP.

To investigate whether mAb 14G12-5 is useful in detecting DR-70 in ELISA, mAb 14G12-5 was added to ELISA plate as capture antibody for capturing analytes with defined concentrations (0, 0.625, 2.5, 5 and 10 $\mu$g/ml DR-70 provided by AMDL-ELISA DR70®), and HRP-conjugated mAb 31H11-2 was employed as detection antibody for detecting the analytes captured by mAb 14G12-5. The result indicated that the combination of mAb 14G12-5 (48 ng/well) and mAb 31H11-2 (24 ng/well) was useful in detecting DR-70 in ELISA, in which the testing curve generated by plotting the mean absorbance (Y-axis) against different DR-70 concentrations (X-axis) was close to the linear standard curve established by AMDL-ELISA DR70® (data not shown); the data demonstrated that mAb 14G12-5 exhibited a high degree of accuracy in detecting DR-70.

Then, the combination of mAb 14G12-5 and mAb 31H11-2 (respectively serving as capture and detection antibodies in ELISA) was used to detect DR-70 in serum sample. The serum samples obtained from human subjects were serially diluted (0-, 1-, 2- or 3-fold dilution) and the subjected to ELISA analysis. The data demonstrated that mAb 14G12-5 and mAb 31H11-2 were capable of detecting DR-70 at different dilutions (0-, 1-, 2- or 3-fold dilution; data not shown). Further, compared to the polyclonal anti-DR-70 antibody (pAb) as provided in AMDL-ELISA DR70®, the present mAb 14G12-5 exhibited a higher level of accuracy in detecting low concentration of DR-70.

### Example 2 Comparative effects between the present mAb 14G12-5 and polyclonal antibody in detecting serum DR-70

In this example, the efficacy of the present mAb 14G12-5 in detecting serum DR-70 was compared with that of a DR-70 polyclonal antibody, which was raised in house by our team. To this purpose, serum samples were collected from 10 subjects with written consent. Among the 10 subjects, subjects #6 to #10 respectively exhibited serum level of carcinoembryonic antigen (CEA) greater than 8 ng/mL. Each serum sample was serially diluted and subjected to sandwich ELISA analysis, in which the primary antibody was mAb 14G12-5 or DR-70 polyclonal antibody, and the secondary antibody was horseradish peroxidase (HRP) conjugated antibody. Results are provided in Table 5.

Table 5

| | Binding Activity ($\mu$g/mL) | | | Average | |
|---|---|---|---|---|---|
| Subject # | mAb 14G12-5 | Polyclonal | Standard Deviation (STD) | | coefficient of variation, (CV%)* |
| 1 | 0.33 | 0.231 | 0.07 | 0.2805 | 24.96 |
| 2 | 2.183 | 1.912 | 0.191 | 2.05 | 9.36 |
| 3 | 0.209 | 0.248 | 0.028 | 0.229 | 12.07 |
| 4 | 1.064 | 1.111 | 0.033 | 1.088 | 3.056 |
| 5 | 0.975 | 0.961 | 0.01 | 0.968 | 1.022 |
| 6 | 3.274 | 3.534 | 0.184 | 3.404 | 5.401 |
| 7 | 5.849 | 4.084 | 1.248 | 4.967 | 25.13 |
| 8 | 1.033 | 0.986 | 0.033 | 1.01 | 3.292 |
| 9 | 0.572 | 0.587 | 0.011 | 0.58 | 1.830 |
| 10 | 0.458 | 0.662 | 0.144 | 0.56 | 25.76 |
| *: CV% = standard deviation/average * 100%, the variation between groups of data was acceptable when CV% < 30%. | | | | | |

It is evident from Table 5, both the DR-70 polyclonal antibody and the present mAb 14G12-5 were effective in detecting DR-70 in serum samples, as the detection efficacy of the polyclonal antibody did not vary far from that of the present monoclonal antibody (CV% < 30%).

In conclusion, the present disclosure provides a novel monoclonal antibody 14G12-5. According to working example of the present disclosure, the antibody14G12-5 exhibits binding affinity to human FDP and DR-70, and accordingly, may serve as an antibody (*e.g.*, a capture or detection antibody) in DR-70 immunoassay for detecting DR-70 or diagnosing cancers.

It will be understood that the above description of embodiments is given by way of example only and that various modifications may be made by those with ordinary skill in the art. The above specification, examples and data provide a complete description of the structure and use of exemplary embodiments of the invention. Although various embodiments of the invention have been described above with a certain degree of particularity, or with reference to one or more individual embodiments, those with ordinary skill in the art could make numerous alterations to the disclosed embodiments without departing from the spirit or scope of this invention.

**Claims**

1. A recombinant antibody or a fragment thereof comprising a heavy chain variable (VH) domain and a light chain variable (VL) domain, wherein

   the VH domain comprises a first complementarity determining region (CDR-H1) of SEQ ID NO: 1, a second complementarity determining region (CDR-H2) of SEQ ID NO: 2, and a third complementarity determining region (CDR-H3) of SEQ ID NO: 3, and
   the VL domain comprises a first complementarity determining region (CDR-L1) of SEQ ID NO: 5, a second complementarity determining region (CDR-L2) of SEQ ID NO: 6, and a third complementarity determining region (CDR-L3) of SEQ ID NO: 7.

2. The recombinant antibody or a fragment thereof of claim 1, wherein the VH domain has the amino acid sequence of SEQ ID NOs: 4; and the VL domain has the amino acid sequence of SEQ ID NO: 8.

3. The recombinant antibody or the fragment thereof according to claim 1, wherein the fragment of the VH domain has the amino acid sequence of SEQ ID NOs: 9, 10, 11, or 12.

4. The recombinant antibody or the fragment thereof according to claim 1, wherein the fragment of the VL domain has the amino acid sequence of SEQ ID NOs: 13, 14, 15, or 16.

5. An *in vitro* method of making a diagnosis on whether a subj ect has a cancer comprising mixing a biological sample isolated from the subject with the recombinant antibody of claim 1 or the fragment thereof, so as to react DR-70 in the biological sample with the recombinant antibody of claim 1 or the fragment thereof thereby forming a complex in an immunological assay, in which the formation of the complex is an indication that the subject has the cancer.

6. The *in vitro* method of claim 5, further comprising adding a secondary antibody to detect the formed complex, wherein the secondary antibody comprises a VH domain of SEQ ID NO: 20 and a VL domain of SEQ ID NO: 24.

7. The *in vitro* method of claim 5, wherein the cancer is lung cancer, breast cancer, gastric cancer, colorectal cancer, pancreatic cancer, tongue cancer, or hepatocellular carcinoma (HCC).

8. The *in vitro* method of claim 5, wherein the biological sample is selected from the group consisting of a skin biopsy sample, a whole blood sample, a plasma sample, a serum sample, a urine sample, a mucus sample, and purified or filtered forms thereof.

9. The *in vitro* method of claim 8, wherein the biological sample is a serum sample.

10. The *in vitro* method of claim 5, wherein the immunological assay is enzyme-linked immunosorbent assay (ELISA).

11. The *in vitro* method of claim 5, wherein the subject is a human.

Europäisches
Patentamt

European
Patent Office

Office européen
des brevets

# EUROPEAN SEARCH REPORT

**Application Number**

EP 24 21 2975

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | ALEXANDER E. KOGAN ET AL: "Monoclonal antibodies with equal specificity to D-dimer and high-molecular-weight fibrin degradation products", BLOOD COAGULATION & FIBRINOLYSIS, vol. 27, no. 5, 11 December 2015 (2015-12-11), pages 542-550, XP055405261, US ISSN: 0957-5235, DOI: 10.1097/MBC.0000000000000453 * page 543, right-hand column * ----- | 1-11 | INV. C07K16/30 C07K16/18 G01N33/50 |
| X | GAFFNEY P. J. ET AL: "Monoclonal antibodies to crosslinked fibrin degradation products (XL-FDP) I. CHARACTERIZATION AND PRELIMINARY EVALUATION IN PLASMA", BRITISH JOURNAL OF HAEMATOLOGY, vol. 68, no. 1, 1 January 1988 (1988-01-01), pages 83-90, XP093273689, Hoboken, USA ISSN: 0007-1048, DOI: 10.1111/j.1365-2141.1988.tb04183.x * page 83, paragraph summary * ----- | 1-11 | |

TECHNICAL FIELDS SEARCHED (IPC)

C07K

-/--

The present search report has been drawn up for all claims

| Place of search | Date of completion of the search | Examiner |
|---|---|---|
| The Hague | 30 April 2025 | Siaterli, Maria |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

**EUROPEAN SEARCH REPORT**

**Application Number**

EP 24 21 2975

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | MCCABE RICHARD P ET AL: "A Diagnostic-Prognostic Test for Bladder Cancer Using a Monoclonal Antibody-based Enzyme-linked Immunoassay for Detection of Urinary Fibrin(ogen) Degradation Products1", CANCER RESEARCH, vol. 44, 1 December 1984 (1984-12-01), XP093273835, Retrieved from the Internet: URL:https://watermark.silverchair.com/cr04 412p15886.pdf?token=AQECAHi208BE49Ooan9kkh W_Ercy7Dm3ZL_9Cf3qfKAc485ysgAAA3UwggNxBgkq hkiG9w0BBwagggNiMIIDXgIBADCCA1cGCSqGSIb3DQ EHATAeBglghkgBZQMEAS4wEQQM3k3-zgDzWTkJipWt AgEQgIIDKK0XiUkHb4UMJIXPqxgHgmw1WECDtNhI6Z PUaIeTPtyYrXWiwFUfuS71RfSWf64U3T8TJbmOvXEM FJv4T81KsD> * abstract * * page 5887, left-hand column, paragraph 3 * * page 5887, right-hand column, paragraph 4 * ----- | 1-11 | |
| A | Amdl ET AL: "501(k) summary AMDL-ELISA DR-70 (R) (FDP) - FDA application", , 1 July 2008 (2008-07-01), XP093273690, Retrieved from the Internet: URL:https://www.accessdata.fda.gov/cdrh_do cs/pdf7/K072901.pdf?utm * the whole document * ----- | 5-11 | TECHNICAL FIELDS SEARCHED (IPC) |

The present search report has been drawn up for all claims

| Place of search | Date of completion of the search | Examiner |
|---|---|---|
| The Hague | 30 April 2025 | Siaterli, Maria |

**EP 4 567 047 A1**

**Non-patent literature cited in the description**

- **ABBAS et al.** Cellular and Molecular Immunology. 2000

- **KABAT et al.** Sequences of Proteins of Immunological Interest. National Institute of Health, 1991